# FASCICULE DE BREVET EUROPEEN

(11) **EP 2 922 785 B1**
(45) Date de publication et mention de la délivrance du brevet: **27.06.2018**
(21) Numéro de dépôt: 13815036.2
(22) Date de dépôt: 19.11.2013
(51) Int. Cl.: C01B 21/093, C07C 303/40

(54) **PROCEDE DE PREPARATION DE SEL D'IMIDES CONTENANT UN GROUPEMENT FLUOROSULFONYLE**
VERFAHREN ZUR HERSTELLUNG VON IMIDSALZEN MIT EINER FLUORSULFONYLGRUPPE
METHOD FOR PREPARING IMIDE SALTS CONTAINING A FLUOROSULPHONYL GROUP

(30) Priorité: 22.11.2012 FR 1261127
(43) Date de publication de la demande: 30.09.2015
(60) Demande divisionnaire: 18172111.9
(73) Titulaire: Arkema France, 92700 Colombes (FR)
(72) Inventeur: AUDUREAU, Sophie, 69320 Feyzin (FR); SCHMIDT, Grégory, 69440 Mornant (FR)
(74) Mandataire: Chahine, Audrey Claire
(86) Numéro de dépôt international: PCT/FR2013/052785
(87) Numéro de publication internationale: WO 2014/080120

(56) Documents cités:
- EP-A1- 2 674 395
- WO-A1-02/053494
- WO-A1-2012/108284
- WO-A1-2012/108384
- WO-A2-2007/068822
- CN-A- 101 654 229

## Description

### DOMAINE DE L'INVENTION

La présente invention concerne un procédé de fluoration pour l'obtention de composés fluorés comprenant au moins un groupement fluorosulfonyle.

### ARRIERE-PLAN TECHNIQUE

Les anions de type sulfonyl imide, de part leur très faible basicité sont de plus en plus dans le domaine du stockage d'énergie sous forme de sels inorganiques dans les batteries ou de sels organiques dans les super condensateurs ou dans le domaine des liquides ioniques. Le marché des batteries étant en plein essor et la réduction des coûts de fabrication des batteries devenant un enjeu majeur, un procédé de synthèse grande échelle et bas coût de ce type d'anions est nécessaire.

Dans le domaine spécifique des batteries Li-ion, le sel actuellement le plus utilisé est le LiPF₆ mais ce sel montre de nombreux désavantages tels qu'une stabilité thermique limité, une instabilité à l'hydrolyse et donc une plus faible sécurité de la batterie. Récemment de nouveaux sels possédant le groupement FSO₂⁻ ont été étudiés et ont démontré de nombreux avantages comme une meilleure conductivité ionique et une résistance à l'hydrolyse. L'un de ces sels le LiFSI(LiN(FSO₂)₂) a montré des propriétés très intéressantes qui font de lui un bon candidat pour remplacer le LiPF₆.

Peu de procédé de synthèse du LiFSI ou de son acide correspondant ont été décrits, mais il apparait clairement que dans tous ces procédés l'étape clé est l'étape de formation de la liaison S-F.

La première voie de synthèse décrite (Appel & Eisenbauer, Chem Ber. 95, 246-8, 1962) consiste en la réaction de l'acide fluorosulfonique (FSO₃H) avec de l'urée. L'utilisation de FSO₃H permettant ainsi d'avoir déjà la liaison S-F formée mais le caractère corrosif et toxique de ce produit ne permettait pas une industrialisation du procédé.

Une seconde voie (Ruff & Lustig, Inorg. Synth. 1968, 11, 138_43) consiste à synthétiser dans un premier temps un composé dichloré de formule suivante (ClSO₂)₂NH puis de réaliser l'échange chlore/fluor avec de l'AsF₃. Mais ce procédé n'est pas industrialisable du fait du prix élevé et de la toxicité de l'AsF₃.

Le document WO02/053494 décrit une troisième voie qui consiste en un échange Cl/F sur le (ClSO₂)₂NH à l'aide d'un fluorure de cation monovalent qui peut être alcalin ou de type onium (NR₄⁺) dans un solvant aprotique. D'après ce document, la réaction s'avère très lente.

L'exemple 10 du document WO07068822 décrit la synthèse du bis(fluorosulfonyl)imide dans l'acide fluorhydrique anhydre. Ainsi, la réaction est mise en oeuvre dans un autoclave avec 1 g du bis(chlorosulfonyl)imide et 4 g de HF anhydre à différentes températures et durée de réaction. Ce document enseigne que même à des températures de 130°C, le rendement de la réaction ne dépasse pas 55%. En outre, il enseigne que la présence d'impuretés rend la séparation difficile à l'échelle industrielle. Ce document conclue que la synthèse du bis(fluorosulfonyl)imide par l'HF n'est pas satisfaisante et recommande l'emploi d'un fluorure de lithium.

Malgré ce préjugé, la demanderesse a mis au point un procédé de fabrication de composés fluorés comprenant au moins un groupement fluorosulfonyle (incluant le bis(fluorosulfonyl)imide) en mettant en jeu l'acide fluohydrique anhydre. Ce procédé présente l'avantage d'être facilement extrapolable à l'échelle industrielle et l'HF présente également l'avantage d'être peu coûteux.

### DESCRIPTION DE L'INVENTION

La demanderesse a observé de manière surprenante qu'en faisant réagir un composé de formule (I) - R₁(SO₂)₂ClNM avec de l'acide fluorhydrique anhydre dans un solvant organique, le rendement en composé fluoré est quasi quantitatif.

Le procédé de préparation de composés fluorés, selon la présente invention, comprend au moins une étape de réaction d'un composé de formule (I) avec de l'acide fluorhydrique anhydre dans au moins un solvant organique. L'étape de réaction avec de l'acide fluorhydrique anhydre, selon la présente invention, peut être schématisé comme suit : 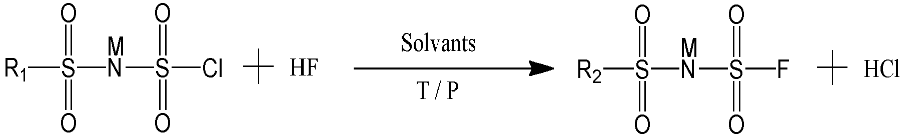 dans lequel :
- R₁ est égal à R₂ sauf dans le cas particulier où R₁ = CI alors R₂ = F. Lorsque R₁ est égal à R₂, R₁ et R₂ représentent un groupement électro-attracteur qui a un paramètre σₚ de Hammett supérieur à 0 tels que F, CF₃, CHF₂, CH₂F, C₂HF₄, C₂H₂F₃, C₂H₃F₂, C₂F₅, C₃F₇, C₃H₂F₅, C₃H₄F₃, C₃HF₆, C₄F₉, C₄H₂F₇, C₄H₄F₅, C₅F₁₁, C₃F₅OCF₃, C₂F₄OCF₃, C₂H₂F₂OCF₃, CF₂OCF₃, C₆F₁₃, C₇F₁₅, C₈F₁₇ OU C₉F₁₉ :
- M représente un atome d'hydrogène, un métal alcalin, alcalino-terreux ou un cation ammonium quaternaire.

Le solvant organique, de préférence, possède un nombre donneur compris entre 1 et 70 avantageusement compris entre 5 et 65. L'indice donneur d'un solvant représente la valeur -ΔH, ΔH étant l'enthalpie de l'interaction entre le solvant et le pentachlorure d'antimoine (Journal of Solution Chemistry, vol. 13, No. 9, 1984). Comme solvant, on peut citer notamment les esters, les nitriles ou dinitriles, les éthers ou diéthers, les amines ou les phosphines.

L'acétate de méthyle, l'acétate d'éthyle, l'acétate de butyle, l'acétonitrile, le propionitrile, l'isobutyronitrile, le glutaronitrile, le dioxane, le tétrahydrofurane, la triéthylamine, la tripropylamine, la diéthylisopropylamine, la pyridine, la triméthylphosphine, la triéthylphosphine et la diéthylisopropylphosphine peuvent convenir comme solvants.

L'étape de réaction avec de l'acide fluorhydrique anhydre peut être mise en oeuvre à une température T, de préférence comprise entre 0°C et la température d'ébullition du solvant ou du mélange de solvants utilisé. Avantageusement, cette température est comprise entre 5°C et la température d'ébullition du solvant ou du mélange de solvants.

Selon la présente invention, l'étape de réaction avec de l'acide fluorhydrique anhydre peut être mise en oeuvre à une pression P, de préférence comprise entre 0 et 16 bars absolus.

Le procédé, selon la présente invention, est de préférence mise en oeuvre en dissolvant le composé de formule (I) dans le solvant ou le mélange de solvants préalablement à l'étape de réaction avec de l'HF anhydre.

Le rapport massique entre le composé de formule(I) et le solvant ou le mélange de solvants est de préférence compris entre 0,001 et 10 et avantageusement compris entre 0,005 et 5.

L'HF est introduit dans le milieu réactionnel, de préférence sous forme gazeuse.

Le rapport molaire entre le composé de formule (I) et l'HF mis en jeu est de préférence compris entre 0,01 et 0,5 et avantageusement compris entre 0,05 et 0,5.

L'étape de réaction avec l'HF peut être effectuée en milieu fermé ou en milieu ouvert.

Sans être liée par une explication, l'utilisation d'un solvant donneur permet la formation d'un complexe solvant-HF et ainsi d'exalter la nucléophilie de l'atome de fluor. L'utilisation d'un tel complexe permet une fluoration douce du composé de formule (I) en évitant ainsi les réactions parasites de coupure.

Le procédé selon la présente invention permet d'avoir des rendements de fluoration compris entre 85 et 100% qui est une nette augmentation comparée aux procédés de l'art antérieur.

Lorsque M dans la formule (I) est égal à H, le procédé selon la présente invention peut comprendre une étape d'échange de cation après l'étape de fluoration pour obtenir des sels de métaux alcalins, alcalino-terreux ou de cations ammonium quaternaires.

Le procédé selon la présente invention est particulièrement intéressant pour préparer de composés fluorés de formules suivantes : LiN(FSO₂)₂, LiNSO₂CF₃SO₂F, LiNSO₂C₂F₅SO₂F, LiNSO₂CF₂OCF₃SO₂F, LiNSO₂C₃HF₆SO₂F, LiNSO₂C₄F₉SO₂F, LiNSO₂C₅F₁₁SO₂F, LiNSO₂C₆F₁₃SO₂F, LiNSO₂C₇F₁₅SO₂F, LiNSO₂C₈F₁₇SO₂F et LiNSO₂C₉F₁₉SO₂F.

La présente invention est illustrée par les exemples suivants, auxquels elle n'est cependant pas limitée.

### Exemple 1 :

Dans un autoclave de 800 mL, 28 g de (ClSO₂)₂NH sont dissous dans 50 mL d'acétonitrile. 10 g d'HF sont ensuite additionnés. La pression est alors de 0,34 bar absolu et la température est maintenue à 10°C. La réaction est laissée sous agitation en milieu fermé pendant 18h. L'HF, en excès, est éliminé par pompage. Le milieu réactionnel est ensuite traité avec du carbonate de lithium. La solution est filtrée puis évaporé et le résidu est analysé par RMN ¹⁹F. L'analyse montre la présence de 85 % de produit totalement fluoré (FSO₂)₂NLi, 7,5% de FSO₃Li et 7,5% de FSO₂NH₂. Ces deux derniers sont les composés formés lors de la dégradation du produit de départ.

### Exemple 2 :

Dans un autoclave de 800 mL, 31,7 g de (ClSO₂)₂NH sont dissous dans 50 mL d'acétonitrile. 10 g d'HF sont ensuite additionnés. La pression est alors de 0,75 bar absolu et la température est maintenue à 20°C. La réaction est laissée sous agitation en milieu fermé pendant 18 h. L'HF, en excès, est éliminé par pompage. Le milieu réactionnel est ensuite traité avec du carbonate de lithium. La solution est filtrée puis évaporé et le résidu est analysé par RMN ¹⁹F. L'analyse montre la présence de 100 % de produit totalement fluoré (FSO₂)₂NLi et l'absence des produits de dégradation FSO₃Li et FSO₂NH₂.

### Exemple 3 :

Dans un autoclave de 800 mL, 61 g de (ClSO₂)₂NH sont dissous dans 50 mL de 1,4-dioxane. 20 g d'HF sont ensuite additionnés. La pression est alors de 2,3 bars absolus et la température est maintenue à 25°C. La réaction est laissée sous agitation en milieu fermé pendant 18 h. L'HF, en excès, est éliminé par pompage. Le milieu réactionnel est ensuite traité avec du carbonate de lithium. La solution est filtrée puis évaporé et le résidu est analysé par RMN ¹⁹F. L'analyse montre la présence de 100 % de produit totalement fluoré (FSO₂)₂NLi et l'absence des produits de dégradation FSO₃Li et FSO₂NH₂.

## Revendications

1. Procédé de préparation d'un composé fluoré de formule (II) comprenant au moins une étape de réaction d'un composé de formule (I) avec de l'acide fluorhydrique anhydre dans au moins un solvant organique selon le schéma suivant : 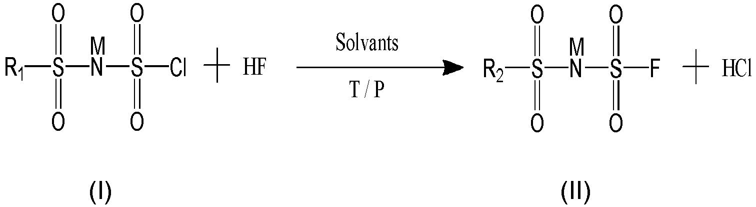 dans lequel :
R₁ est égal à R₂ sauf dans le cas particulier où R₁ = Cl alors R₂ = F₁ et lorsque R₁ est égal à R₂, R₁ et R₂ représentent un groupement électro-attracteur qui a un paramètre σₚ de Hammett supérieur à 0 tels que F, CF₃, CHF₂, CH₂F, C₂HF₄, C₂H₂F₃, C₂H₃F₂, C₂F₅, C₃F₇, C₃H₂F₅, C₃H₄F₃, C₃HF₆, C₄F₉, C₄H₂F₇, C₄H₄F₅, C₅F₁₁, C₃F₅OCF₃, C₂F₄OCF₃, C₂H₂F₂OCF₃, CF₂OCF₃, C₆F₁₃, C₇F₁₅, C₈F₁₇ ou C₉F₁₉ et M représente un atome d'hydrogène, un métal alcalin, alcalino-terreux ou un cation ammonium quaternaire.

2. Procédé selon la revendication 1, dans lequel le solvant organique, possède un nombre donneur compris entre 1 et 70 avantageusement compris entre 5 et 65.

3. Procédé selon la revendication 1 ou 2, dans lequel le solvant peut être choisi parmi les esters, les nitriles ou dinitriles, les éthers ou diéthers, les amines ou les phosphines.

4. Procédé selon l'une des revendications 1 à 3, **caractérisé en ce que** l'étape de réaction est mise en oeuvre à une température T comprise entre 0°C et la température d'ébullition du solvant ou du mélange de solvants, de préférence comprise entre 5°C et la température d'ébullition du solvant ou du mélange de solvants.

5. Procédé selon l'une des revendications 1 à 4 **caractérisé en ce que** l'étape de réaction est mise en oeuvre à une pression P comprise entre 0 et 16 bars absolus.

6. Procédé selon l'une des revendications 1 à 5 **caractérisé en ce que** le composé de formule (I) est dissout dans le solvant ou le mélange de solvants préalablement à l'étape de réaction avec de l'HF anhydre.

7. Procédé selon l'une des revendications 1 à 6 **caractérisé en ce que** le rapport massique entre le composé de formule(I) et le solvant ou le mélange de solvants est compris entre 0,001 et 10 et de préférence compris entre 0,005 et 5.

8. Procédé selon l'une des revendications 1 à 7 **caractérisé en ce que** le rapport molaire entre le composé de formule(I) et l'HF mis en jeu est compris entre 0,01 et 0,5 et de préférence compris entre 0,05 et 0,5.

9. Procédé selon l'une des revendications 1 à 8 **caractérisé en ce qu'**il comprend une étape d'échange de cation après l'étape de fluoration pour obtenir des sels de métaux alcalins, alcalino-terreux ou de cations ammonium quaternaires.

10. Procédé selon l'une des revendications 1 à 9 **caractérisé en ce que** le composé de formule (II) est le LiN(FSO₂)₂), LiNSO₂CF₃SO₂F, LiNSO₂C₂F₅SO₂F, LiNSO₂CF₂OCF₃SO₂F, LiNSO₂C₃HF₆SO₂F, LiNSO₂C₄F₉SO₂F, LiNSO₂C₅F₁₁SO₂F, LiNSO₂C₆F₁₃SO₂F, LiNSO₂C₇F₁₅SO₂F, LiNSO₂C₈F₁₇SO₂F et LiNSO₂C₉F₁₉SO₂F.

## Patentansprüche

1. Verfahren zur Herstellung einer Fluorverbindung der Formel (II), umfassend mindestens einen Schritt der Umsetzung einer Verbindung der Formel (I) mit wasserfreier Fluorwasserstoffsäure in mindestens einem organischen Lösungsmittel gemäß dem folgenden Schema: 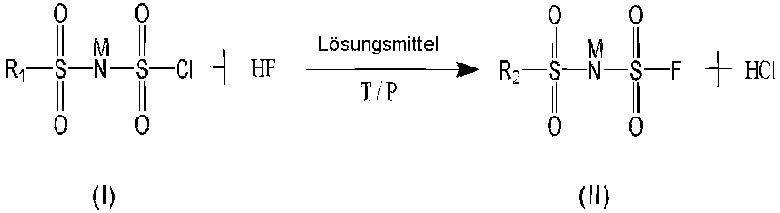 wobei:
R₁ gleich R₂ ist, außer in dem speziellen Fall, in dem R₁ = Cl, wobei dann R₂ = F₁ ist, und dann, wenn R₁ gleich R₂ ist, R₁ und R₂ für eine elektronenanziehende Gruppe mit einem Hammett-Parameter σₚ von mehr als 0, wie F, CF₃, CHF₂, CH₂F, C₂HF₄, C₂H₂F₃, C₂H₃F₂, C₂F₅, C₃F₇, C₃H₂F₅, C₃H₄F₃, C₃HF₆, C₄F₉, C₄H₂F₇, C₄H₄F₅, C₅F₁₁, C₃F₅OCF₃, C₂F₄OCF₃, C₂H₂F₂OCF₃, CF₂OCF₃, C₆F₁₃, C₇F₁₅, C₈F₁₇ oder C₉F₁₉, stehen und M für ein Wasserstoffatom, ein Alkalimetall, ein Erdalkalimetall oder ein quaternäres Ammoniumkation steht.

2. Verfahren nach Anspruch 1, bei dem das organische Lösungsmittel eine Donorzahl zwischen 1 und 70 und vorteilhafterweise zwischen 5 und 65 aufweist.

3. Verfahren nach Anspruch 1 oder 2, wobei das Lösungsmittel aus Estern, Nitrilen oder Dinitrilen, Ethern oder Diethern, Aminen oder Phosphinen ausgewählt werden kann.

4. Verfahren nach einem der Ansprüche 1 bis 3, **dadurch gekennzeichnet, dass** der Umsetzungsschritt bei einer Temperatur T zwischen 0 °C und dem Siedepunkt des Lösungsmittels oder Lösungsmittelgemischs, vorzugsweise zwischen 5 °C und dem Siedepunkt des Lösungsmittels oder Lösungsmittelgemischs, durchgeführt wird.

5. Verfahren nach einem der Ansprüche 1 bis 4, **dadurch gekennzeichnet, dass** der Umsetzungsschritt bei einem Druck P zwischen 0 und 16 bar absolut durchgeführt wird.

6. Verfahren nach einem der Ansprüche 1 bis 5, **dadurch gekennzeichnet, dass** die Verbindung der Formel (I) vor dem Schritt der Umsetzung mit dem wasserfreien HF in dem Lösungsmittel oder Lösungsmittelgemisch gelöst wird.

7. Verfahren nach einem der Ansprüche 1 bis 6, **dadurch gekennzeichnet, dass** das Massenverhältnis zwischen der Verbindung der Formel (I) und dem Lösungsmittel oder Lösungsmittelgemisch zwischen 0,001 und 10 und vorzugsweise zwischen 0,005 und 5 liegt.

8. Verfahren nach einem der Ansprüche 1 bis 7, **dadurch gekennzeichnet, dass** das verwendete Molverhältnis zwischen der Verbindung der Formel (I) und dem HF zwischen 0,01 und 0,5 und vorzugsweise zwischen 0,05 und 0,5 liegt.

9. Verfahren nach einem der Ansprüche 1 bis 8, **dadurch gekennzeichnet, dass** es einen Ionenaustauschschritt nach dem Fluorierungsschritt zum Erhalt von Alkalimetallsalzen, Erdalkalimetallsalzen oder Salzen quaternärer Ammoniumkationen umfasst.

10. Verfahren nach einem der Ansprüche 1 bis 9, **dadurch gekennzeichnet, dass** es sich bei der Verbindung der Formel (II) um LiN(FSO₂)₂), LiNSO₂CF₃SO₂F, LiNSO₂C₂F₅SO₂F, LiNSO₂CF₂OCF₃SO₂F, LiNSO₂C₃HF₆SO₂F, LiNSO₂C₄F₉SO₂F, LiNSO₂C₅F₁₁SO₂F, LiNSO₂C₆F₁₃SO₂F, LiNSO₂C₇F₁₅SO₂F, LiNSO₂C₈F₁₇SO₂F und LiNSO₂C₉F₁₉SO₂F handelt.

## Claims

1. Process for preparing a fluorinated compound of formula (II), comprising at least one step of reacting a compound of formula (I) with anhydrous hydrofluoric acid in at least one organic solvent according to the following scheme: 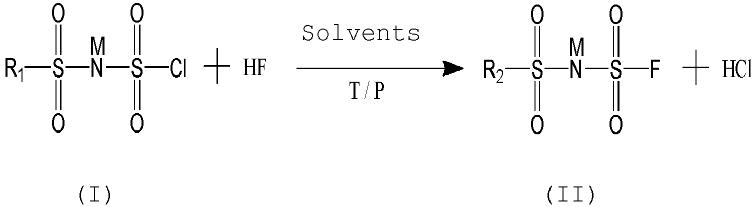 in which:
R₁ is equal to R₂ except in the specific case where R₁ = Cl, then R₂ = F₁, and where R₁ is equal to R₂, R₁ and R₂ represent an electron-withdrawing group which has a Hammett parameter σₚ greater than 0, such as F, CF₃, CHF₂, CH₂F, C₂HF₄, C₂H₂F₃, C₂H₃F₂, C₂F₅, C₃F₇, C₃H₂F₅, C₃H₄F₃, C₃HF₆, C₄F₉, C₄H₂F₇, C₄H₄F₅, C₅F₁₁, C₃F₅OCF₃, C₂F₄OCF₃, C₂H₂F₂OCF₃, CF₂OCF₃, C₆F₁₃, C₇F₁₅, C₈F₁₇ or C₉F₁₉, and M represents a hydrogen atom, an alkali metal, an alkaline-earth metal or a quaternary ammonium cation.

2. Process according to Claim 1, wherein the organic solvent has a donor number of between 1 and 70, advantageously between 5 and 65.

3. Process according to Claim 1 or 2, wherein the solvent may be chosen from esters, nitriles or dinitriles, ethers or diethers, amines or phosphines.

4. Process according to one of Claims 1 to 3, **characterized in that** the reaction step is carried out at a temperature T of between 0°C and the boiling point of the solvent or of the mixture of solvents, preferably between 5°C and the boiling point of the solvent or of the mixture of solvents.

5. Process according to one of Claims 1 to 4, **characterized in that** the reaction step is carried out at a pressure P of between 0 and 16 bar absolute.

6. Process according to one of Claims 1 to 5, **characterized in that** the compound of formula (I) is dissolved in the solvent or the mixture of solvents prior to the step of reacting with anhydrous HF.

7. Process according to one of Claims 1 to 6, **characterized in that** the weight ratio between the compound of formula (I) and the solvent or the mixture of solvents is between 0.001 and 10 and preferably between 0.005 and 5.

8. Process according to one of Claims 1 to 7, **characterized in that** the molar ratio between the compound of formula (I) and the HF used is between 0.01 and 0.5 and preferably between 0.05 and 0.5.

9. Process according to one of Claims 1 to 8, **characterized in that** it comprises a cation exchange step after the fluorination step in order to obtain alkali metal salts, alkaline-earth metal salts or quaternary ammonium cation salts.

10. Process according to one of Claims 1 to 9, **characterized in that** the compound of formula (II) is LiN(FSO₂)₂, LiNSO₂CF₃SO₂F, LiNSO₂C₂F₅SO₂F, LiNSO₂CF₂OCF₃SO₂F, LiNSO₂C₃HF₆SO₂F, LiNSO₂C₄F₉SO₂F, LiNSO₂C₅F₁₁SO₂F, LiNSO₂C₆F₁₃SO₂F, LiNSO₂C₇F₁₅SO₂F, LiNSO₂C₈F₁₇SO₂F or LiNSO₂C₉F₁₉SO₂F.
